# EUROPEAN PATENT APPLICATION

(11) **EP 4 502 143 A1**
(43) Date of publication of application: **05.02.2025**
(21) Application number: 22935899.9
(22) Date of filing: 11.11.2022
(51) Int. Cl.: C12N 5/071, A61K 35/38, A61P 1/04

(54) **MEDIUM COMPOSITION FOR PREPARING INTESTINAL ORGANOID**

(30) Priority: 30.03.2022 KR 20220039992
(71) Applicant: Organoidsciences Ltd., Daejeon 34141 (KR)
(72) Inventor: LEE, Hyokyung, Seongnam-si Gyeonggi-do 13488 (KR); CHOI, Soohee, Seongnam-si Gyeonggi-do 13488 (KR); KANG, Eunseo, Seongnam-si Gyeonggi-do 13488 (KR); TAK, Ji Hye, Seongnam-si Gyeonggi-do 13488 (KR); PARK, Jun-Hyeok, Seongnam-si Gyeonggi-do 13488 (KR); KIM, Yong-Il, Seongnam-si Gyeonggi-do 13488 (KR)
(74) Representative: Rückerl, Florian
(86) International application number: PCT/KR2022/017750
(87) International publication number: WO 2023/191225

(57) **Abstract**

The present invention relates to a medium composition for preparing an intestinal organoid. In addition, the present invention relates to: a method for preparing an intestinal organoid, comprising a step of culturing using the medium composition; and an intestinal organoid prepared by the preparation method. The medium for preparing an intestinal organoid can promote the proliferation of the intestinal organoid and maintain the sternness of the intestinal organoid. Therefore, the medium can be used in preparing a large amount of intestinal organoids of excellent quality, and intestinal organoids prepared using the medium can be used as agents for preventing or treating intestine-associated diseases, alternatives to animal experimental models of intestine-associated diseases, and the like.

## Description

### [Technical Field]

The present invention relates to a medium composition for preparing an intestinal organoid. In addition, the present invention relates to: a method for preparing an intestinal organoid, comprising a step of culturing using the medium composition; and an intestinal organoid prepared by the preparation method.

### [Background Art]

As stem cells have unique multi-potency and self-renewal characteristics, research for using them in various ways for treatment of incurable diseases, disease modeling, transplantation of tissues or organs, etc. is steadily progressing. In particular, it has been found that when stem cells are cultured in an appropriate three-dimensional laboratory environment, a cell mass having structural and tissue characteristics similar to that of an in vivo organ is formed, and such a cell mass is called an organoid.

Since technology for preparing an organoid can theoretically produce almost all types of organs only with stem cells, it is expected to be used in the development of therapeutic agents for various diseases. It is believed that organoids can be more effective in testing the safety and efficacy of new drugs than cell tissue made in two dimensions, and can be used to improve the condition by transplanting to damaged or underdeveloped organs. Accordingly, from the point of view of regenerative medicine, research on organoids has become more active in recent years, and it is expected that organoids will be widely used in various fields.

However, technology for preparing and culturing organoids is still at an early stage of research, and there is still a need for various research and development on medium compositions specific to each organ and methods for producing organoids using the same, such as establishing the combination or ratio of medium-added components.

### [Technical Problem]

The inventors have conducted various studies to efficiently prepare intestinal organoids with excellent quality. As a result, a specific combination was identified among various media components which capable of maximizing the promotion of proliferation and the maintenance of sternness for intestinal organoids, and the present invention was completed by experimentally demonstrating its effect.

### [Technical Solution]

Each description and embodiment disclosed in the present invention is also applicable to other descriptions and embodiments. That is, all combinations of the various elements disclosed herein fall within the scope of the present invention. In addition, it cannot be considered that the scope of the present invention is limited by the specific descriptions disclosed below.

In addition, it should be understood that terms not specifically defined in this specification have meanings commonly used in the technical field to which the present invention pertains. Also, unless otherwise defined by context, the singular includes the plural, and the plural includes the singular.

One aspect of the present invention provides a medium composition for preparing an intestinal organoid comprising HGF and NRG1.

The medium composition of the present invention can form a large number of intestinal organoids by promoting their proliferation, and allows most of the formed intestinal organoids to maintain stemness. Intestinal organoids prepared by culturing in this way can be applied in various fields such as regenerative therapy, cell therapy, drug screening and drug evaluation.

The term "organoid" as used herein refers to a cell mass with a 3D three-dimensional structure. It is manufactured through an artificial culture process rather than collected, acquired, or harvested from animals, etc., and is defined as a miniaturized and simplified version of the organ. The organoid according to the present invention may be an intestinal organoid, and the intestinal organoid may be a small intestine organoid, a duodenal organoid, a large intestine organoid, a rectal organoid or a colon organoid, but is not limited thereto.

The term "HGF" as used herein refers to a Hepatocyte Growth Factor, and the information on its protein sequence, etc., can be found in a known database (NCBI Reference Sequence: NP_000592, NP_001010931, NP_001010932, NP_001010933, NP_001010934). The HGF may be contained in the medium composition of the present invention at a concentration of 1 to 100 ng/ml, more specifically at a concentration of 50 ng/ml, but is not limited thereto.

The term "NRG1" as used herein refers to a Neuregulin 1 protein encoded by NRG1 gene. The information on its protein sequence, etc., can be found in a known database (NCBI Reference Sequence: NP_001153467, NP_001153468, NP_001153471, NP_001153473, NP_001153474). The NRG1 may be contained in the medium composition of the present invention at a concentration of 50 to 150 ng/ml, more specifically at a concentration of 100 ng/ml, but is not limited thereto.

In the present invention, when the intestinal organoids are cultured with the medium containing HGF and NRG1, it was confirmed that the production effects of intestinal organoids are excellent. For example, the proliferation of the intestinal organoids is promoted, the number of obtainable intestinal organoids is significantly increased, and at the same time, most of the formed intestinal organoids maintain stemness.

Specifically, the intestinal organoid according to the present invention may express Tyrosine-protein kinase-like 7 (PTK7).

The term "Tyrosine-protein kinase-like 7 (PTK7)" as used herein refers to a gene used as a stem cell marker, and intestinal organoids expressing PTK7 are known to show sternness such as high self-renewal and re-seeding ability (Stem Cell Reports. 2015 Dec 8;5(6):979-987).

In addition, the medium composition for preparing an intestinal organoid of the present invention may further comprise IGF1.

The term "IGF1" as used herein refers to insulin like growth factor 1 protein encoded by IGF1 gene. The information on its protein sequence, etc., can be found in a known database (NCBI Reference Sequence: NP_000609.1, NP_001104753.1, NP_001104754.1, NP_001104755.1, XP_016874748.1, XP_016874750.1, XP_016874751.1, XP_016874752.1). The IGF1 may be contained in the medium composition of the present invention at a concentration of 50 to 100 ng/ml, more specifically at a concentration of 100 ng/ml, but is not limited thereto.

The acquisition route of the HGF, NRG1 and IGF1 is not particularly limited. For example, it can be prepared using commercially available materials, or using a gene (protein) recombination technique according to a method known in the art.

In the present invention, the intestinal organoids may be cultured for more than 7 passages, for more than 9 passages, or for more than 15 passages. In addition, the intestinal organoid may be cultured for more than 20 passages.

The term "passage" as used herein refers to replacing culture container or dividing the cell group in a method of culturing successive generations of cells to continuously cultivate cells or organoids in a healthy state for a long period of time. Passage 1 is replacing the culture container once, or dividing the cell group once. Passage 20 is replacing the culture container 20 times, or dividing the cell group 20 times. As the passage increases, a large number of organoids may be obtained, but the culture medium used in the art has a problem in that cells constituting the organoids die as the passage increases, making long-term culture impossible. The medium composition comprising HGF and NRG1 according to the present invention can culture intestinal organoids for a long period of time for more than 20 passages. In this specification, the "passage" may be used interchangeably with "generation".

As used herein, the term "medium" refers to a mixture of nutritional substances that enable the growth, survival, expansion or differentiation of cells or organoids in vitro, and includes any suitable conventional medium used in the art. The culture medium type and culture condition may be selected at the technical level in the art depending on the type of cells or organoids. The medium may be a basal medium for cell culture containing a carbon source, a nitrogen source and trace elements. As specific examples, it may be DMEM (Dulbecco's Modified Eagle's Medium), MEM (Minimal Essential Medium), BME (Basal Medium Eagle), RPMI1640, F-10, F-12, α-MEM (α-Minimal Essential Medium), GMEM (Glasgow's Minimal Essential Medium), IMDM (Iscove's Modified Dulbecco's Medium), DMEM/F12 (Dulbecco's Modified Eagle Medium/Nutrient Mixture F-12) or Advanced DMEM/F12, but is not limited thereto.

The prior art in the art for a method for preparing an organoid uses at least two types of culture media having different components. For example, at the beginning of the culture, a growth medium is used to form and grow organoids, and after the growth medium is removed, an expansion medium is used to increase the number of organoids. However, the conventional method for producing organoids using two or more types of culture media with different components has disadvantages in that it is expensive and the manufacturing process becomes complicated.

In addition, the organoid has a characteristic that the production result is significantly different depending on the culture conditions due to their properties. Culture conditions such as culture day, culture temperature, culture medium and culture substrate are important factors that determine the characteristics of organoids. Among them, medium conditions play the biggest role in growing organoids to have characteristics similar to desired tissues or organs by regulating various signaling pathways in stem cells.

In the present invention, it was confirmed that the specific medium condition comprises HGF and NRG1 for obtaining a sufficient number of intestinal organoids that are very similar in shape and function to the actual tissue, and can be used for regenerative treatment, drug screening, drug evaluation, etc., especially due to increased proliferative capacity and stemness. Furthermore, when using the above culture medium, it was confirmed that the formation, growth and expansion of intestinal organoids could be successfully induced by using only one type of culture medium rather than using two or more types of culture medium.

In this specification, "for preparing" may be used interchangeably with the same meaning as "for culturing". Accordingly, the medium composition for preparing intestinal organoids may be a medium composition for culturing intestinal organoids, a medium composition for forming intestinal organoids, and also a medium composition for expanding intestinal organoids. Herein, the term "for forming" refers to the formation of intestinal organoids in intestine-derived cells, and refers to having organ or tissue characteristics to perform a unique function of the intestine. In addition, the "for expanding" refers to obtaining a sufficient amount that can be used for a desired purpose by increasing the number of formed intestinal organoids, and means that subculture of two or more passages is possible.

In the present invention, the medium composition may additionally comprise an antibiotic, and the antibiotic includes any appropriate conventional antibiotics used for culturing cells or organoids in the art. As specific examples, it may be one or more selected from the group consisting of gentamicin, amphotericin, penicillin and streptomycin, but is not limited thereto.

Another aspect of the present invention provides a method for preparing an intestinal organoid, comprising a step for culturing intestine-derived cells isolated from a subject using the medium composition.

In the method for preparing an intestinal organoid according to the present invention, each term has the same meaning as described in the medium composition for preparing an intestinal organoid, unless otherwise specified.

The term "subject" as used herein may include, without limitation, a human or any non-human animal. The non-human animal may be a vertebrate such as a primate, dog, cow, horse, pig, rodent such as mouse, rat, hamster and guinea pig. In this specification, the "subject" may be used interchangeably with "individual" or "patient".

The isolated intestine-derived cells may be separated through the process of cutting the intestinal tissue of the small or large intestine and the enzymatic digestion process. Specifically, the cutting includes both physical cutting and mechanical cutting, and may be performed by a general tissue cutting method known in the art. In addition, the enzymatic digestion may be performed under general enzymatic digestion conditions known in the art. For example, it may be carried out using one or more enzymes selected from the group consisting of dispase II, DNase I and collagenase II.

In the present invention, the culture may be cultured for more than 20 passages, and one passage may be performed for 1 to 10 days.

Another aspect of the present invention provides an intestinal organoid prepared by the method for preparing an intestinal organoid.

In the intestinal organoid according to the present invention, each term has the same meaning as described in the medium composition for preparing an intestinal organoid, unless otherwise specified.

The intestinal organoid according to the present invention maintains sternness and has a histological composition and function very similar to that of the human intestine. Thus, when administered in vivo, it can be engrafted in the damaged area of the intestine and regenerate it. Accordingly, it can be used as a prophylactic or therapeutic agent for enteropathy, for example an inflammatory bowel disease such as Crohn's disease, ulcerative colitis, intestinal Behcet's disease and proctitis, ulcers or fistula caused by the inflammatory bowel disease, and also as an alternative to animal experimental models of intestine-related diseases.

The term "treatment" or "treating" as used herein refers to any action in which symptoms related to intestine are improved or cured by transplantation or administration of the intestinal organoids according to the present invention. Also, the term "prevention" or "preventing" as used herein refers to any action in which symptoms related to intestine is inhibited or delayed by transplantation or administration of the intestinal organoids according to the present invention.

Another aspect of the present invention provides a pharmaceutical composition for preventing or treating enteropathy, comprising the intestinal organoid.

In the pharmaceutical composition according to the present invention, each term has the same meaning as described in the medium composition for preparing an intestinal organoid, unless otherwise specified.

The pharmaceutical composition according to the present invention may be a composition for regenerative therapeutic agent or cell therapeutic agent.

The term "regenerative therapeutic agent" as used herein refers to a therapeutic agent that restores normal function or creates a new one by replacing or regenerating damaged human cells, tissues or organs. The term "cell therapeutic agent" as used herein refers to a type of a regenerative therapeutic agent, and a drug manufactured for the purpose of prevention, treatment or diagnosis from living autologous, allogeneic or xenogenic cells. The cell therapeutic agent may be used for regeneration of damaged or defective cells or tissues, and may be used to restore the function and shape of damaged or defective cells or tissues.

The pharmaceutical composition according to the present invention may be administered to a subject in need of prevention or treatment of enteropathy by comprising an effective amount of the intestinal organoid.

The enteropathy that can be prevented or treated by the pharmaceutical composition of the present invention may include, for example, one or more selected from the group consisting of inflammatory bowel disease, ulcers due to inflammatory bowel disease, and fistulas due to inflammatory bowel disease, but is not limited to thereto. Also, the inflammatory bowel disease may be one or more selected from the group consisting of radiation colitis, radiation proctitis, ischemic colitis, intestinal Behcet's disease, Crohn's disease, ulcerative colitis and ulcerative proctitis, but is not limited thereto. In addition, the ulcer and fistula are complications caused by inflammatory bowel disease, and the fistula may be one or more selected from the group consisting of anal fistula and enterocutaneous fistula, but is not limited thereto.

The effective amount may be a "therapeutically effective amount" or a "prophylactically effective amount". As used herein, the term "therapeutically effective amount" refers to, when a drug or a therapeutic agent is used alone or in combination with other therapeutic agents, any amount capable of having a decrease in the severity of a disease, an increase in the frequency and duration of the disease symptom-free period, or an inhibition of damage or impairment due to suffering from a disease. In addition, the term "prophylactically effective amount" as used herein refers to any amount to suppress occurrence or recurrence of enteropathy in a subject. The level of effective amount may be determined depending on factors including subject's severity, age, sex, drug activity, drug sensitivity, administration time, administration route and excretion rate, treatment period, concurrent drugs, and other factors well known in the medical field.

As used herein, the term "administration" or "administering" refers to the physical introduction of the composition to a subject using any various methods and delivery systems known to a person skilled in the art. The route of administration for the pharmaceutical composition of the present invention includes, for example, routes of submucosal, intramuscular, subcutaneous, intraperitoneal or spinal administration, or other parenteral routes such as by injection or infusion, but is not limited thereto. In addition, the administration may be non-surgical administration using a device such as a catheter, and also surgical administration methods such as injection or transplantation after incision of the diseased site are possible. The number of administrations for the compositions of the present invention may be, for example, single, multiple, and over one or more extended periods.

The administrations of the composition of the present invention may depend on the age, sex or weight of the subject. Specifically, the pharmaceutical composition may be administered once to several times a day, or administered at intervals of several days to several months, depending on the symptoms of subject. Also, the dosage may be adjusted so that the intestinal organoids contained in the pharmaceutical composition of the present invention are administered at a cell number of 5x105/cm2 to 5x106/cm2, but is not limited thereto. It may increase or decrease depending on the administration route, disease severity, sex, body weight, age, etc.

The pharmaceutical composition of the present invention may further comprise suitable carriers, excipients or diluents commonly used in its preparation. The carriers, excipients or diluents that may be contained in the composition include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil, but are not limited thereto.

The pharmaceutical composition of the present invention may be administered in combination with other therapeutic agents. In this case, the composition of the present invention and other therapeutic agents may be administered simultaneously, sequentially, or separately. The other therapeutic agents may be a drug such as compound and protein which has an effect on prevention, treatment or improvement of enteropathy, but are not limited thereto.

In addition, The pharmaceutical composition of the present invention may be formulated to be administered simultaneously, sequentially or separately with other therapeutic agents. For example, the intestinal organoid and other therapeutic agents may be administered simultaneously as one formulation, or may be administered simultaneously, sequentially or separately as separate formulations. For simultaneous, sequential or separate administration, the intestinal organoid and other therapeutic agents contained in the composition of the present invention may be formulated separately in each container, or may be formulated together in the same container. In addition, the effective amount, administration time, administration interval, administration route, or treatment duration of other therapeutic agents and the intestinal organoid contained in the composition of the present invention may be the same or different from each other.

Another aspect of the present invention provides a method of treating or preventing enteropathy, comprising administering the intestinal organoid to a subject in need of prevention or treatment of enteropathy.

In the method of treating or preventing enteropathy according to the present invention, each term has the same meaning as described in the medium composition for preparing an intestinal organoid and the pharmaceutical composition for preventing or treating enteropathy, unless otherwise specified.

In the method for preventing or treating enteropathy according to the present invention, the intestinal organoid may be administered simultaneously, sequentially or separately with other therapeutic agents to the subject.

The "simultaneous" administration means that the intestinal organoid and other therapeutic agents are administered at once as one formulation. Alternatively, it means administering the intestinal organoids and other therapeutic agents at once as separate preparations, and in this case, the administration routes of the intestinal organoids and other therapeutic agents may be different.

The "sequential" administration means that the intestinal organoid and other therapeutic agents are administered relatively continuously, allowing the minimum possible time as the time consumed in the administration interval.

The "separate" administration means that the intestinal organoid and other therapeutic agents are administered at regular intervals. The administration method of the intestinal organoid and other therapeutic agents may be appropriately selected by doctor or those skilled in the art considering therapeutic effects and side effects of the subject.

The words used in this specification include singular or plural meanings. For example, "ingredient" means one ingredient or more than one ingredient.

The medium for preparing intestinal organoids of the present invention can promote the proliferation of intestinal organoids and maintain the sternness of the intestinal organoids. Therefore, the medium can be utilized to produce a large amount of intestinal organoids of excellent quality, and the intestinal organoids prepared with the medium can be used as a preventive or therapeutic agent for intestinal-related diseases, or an alternative to animal experimental models of intestinal-related diseases.

### [Brief Description of Figures]

FIG. 1 is an image showing the appearance of intestinal organoids prepared with medium of different components. "All" refers to a medium comprising HGF, NRG1 and IGF1; "-HGF" refers to a medium comprising NRG1 and IGF1 except for HGF, "-NRG1" refers to a medium comprising HGF and IGF1 except for NRG1, and "-IGF1" refers to a medium containing HGF and NRG1 except for IGF1 (the same applies to FIG. 2 to FIG. 5).
FIG. 2 is a graph showing the number of intestinal organoids prepared with medium of different components, and relates to short-term culture results within 30 days.
FIG. 3 is a graph showing the number of intestinal organoids prepared with medium of different components, and relates to long-term culture results from 37 to 55 days.
FIG. 4 is a graph showing the sternness of intestinal organoids prepared with medium of different components, and relates to the ratio of intestinal organoids expressing PTK gene. The intestinal organoids were cultured for a total of 55 days (5 passages and 8 days).
FIG. 5 is a graph showing the sternness of intestinal organoids prepared with medium of different components, and relates to the object area of intestinal organoids. A is an image showing the object area of the intestinal organoid, and B is a graph showing the measurement result of the object area shown in A. The intestinal organoids were cultured for a total of 55 days (5 passages and 8 days).
FIG. 6 is an image showing the results of karyotyping, which is one of the genetic stability tests of organoids cultured in a media containing HGF, NRG1 and IGF1 ("All"). The karyotypes were analyzed for organoids cultured at passage 7, passage 9, passage 15 or passage 20.

### [Examples]

Hereinafter, the present invention will be described in more detail by Examples. However, these Examples are only for illustrative purposes, and the scope of the present invention is not limited thereto.

### Example 1. Preparation of a medium composition for preparing intestinal organoids

In order to develop a medium composition for efficiently culturing intestinal organoids, advanced DMEM F/12 was used as a basic medium, and the experimental groups were classified depending on whether HGF, NRG1 or IGF1 is contained, as follows. Herein, HGF was used at 50 ng/ml, NRG1 at 100 ng/ml, and IGF1 at 100 ng/ml.
(1) "All": containing HGF, NRG1 and IGF1
(2) "-HGF": excluding HGF, and containing NRG1 and IGF1
(3) "-NRG1": excluding NRG1, and containing HGF and IGF1
(4) "-IGF1": excluding IGF1, and containing HGF and NRG1

Thereafter, the intestinal tissue of the patient collected through the endoscope or the intestinal tissue obtained through surgery was washed three or more times with a washing solution. The washed intestinal tissue was treated with 1X TrypLE at 37°C for 15 minutes and further treated with 0.1% bovine serum albumin (BSA) mixed with DPBS, and then crypt containing intestinal stem cells was isolated by pipetting. The isolated crypt was passed through a 70 µm cell strainer and centrifuged. And then, the obtained crypt was mixed with the extracellular matrix (collagen) at a 1:1 ratio and cultured in a three-dimensional form for a total of 7 passages for 4 to 7 days per passage.

### Example 2. Results of organoid culture depending on the composition of medium

Through Examples 2-1 to 2-4 below, it was confirmed which component is most effective and essential for culturing organoids among the various components constituting the medium composition.

### Example 2-1. Comparison of the number of organoids prepared

The number of intestinal organoids prepared with each medium composition according to Example 1 was compared. In this Example, it was analyzed whether a sufficient number of organoids are prepared to be used for purposes such as regenerative therapy, drug screening, and drug evaluation.

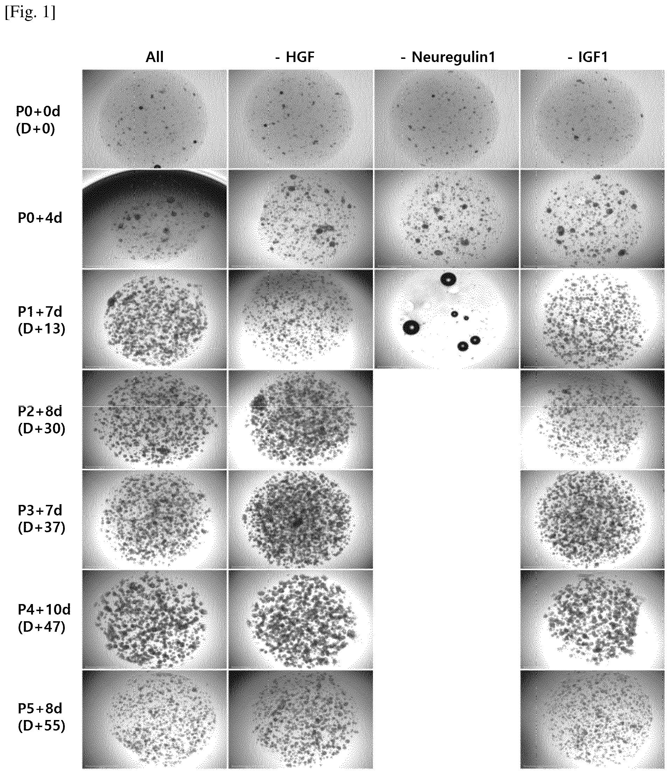

As a result, as shown in FIG. 1 to FIG. 3, it was confirmed that when using a medium not containing NRG1 ("-NRG1"), the organoids grow only for 4 days after the start of the culture, and they are no longer growing and died after 13 days of the culture.

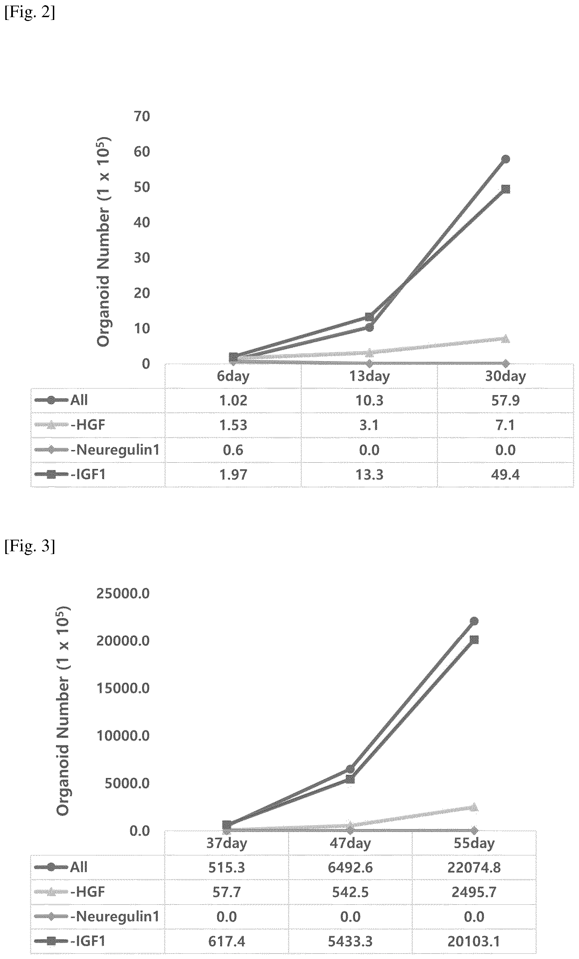

In addition, it was confirmed that when using a medium not containing HGF ("-HGF"), the organoids can grow for a long time up to 55 days (passage 5 and day 8) after the start of culture. However, the number of organoids prepared was reduced to about 1/10 compared to that culturing in the medium containing all components ("All"). Meanwhile, in the case of "-HGF" in the image of FIG. 1, it seems that the number of organoids increases more than the control group "All" from 30 days to 55 days after the start of culture. However, most of the organoids photographed in "-HGF" were dead. The dead and differentiated organoids were washed-out during subculture performed after imaging, and only the number of surviving organoids was measured and graphed (FIG. 2 and FIG. 3).

In addition, it was confirmed that when using a medium not containing IGF1 ("-IGF1"), the organoids can grow for a long time up to 55 days (passage 5 and day 8) after the start of culture, and the number of organoids prepared is similar to that culturing in the medium containing all components ("All").

The above results suggest that NRG1 and HGF are essential components in order to produce a large number of intestinal organoids by promoting the proliferation of intestinal organoids during culture.

### Example 2-2. Comparison of stem cell marker expression levels

In the intestinal organoids prepared with each medium composition according to Example 1, the expression levels of stem cell marker genes were compared. In this Example, it was analyzed whether organoids maintain sternness even in the culture for a long period of time (55 days in total, passage 5 and day 8), and Tyrosine-protein kinase-like 7 (PTK7) was used as a stem cell marker. It is known that intestinal organoids expressing PTK7 have high self-renewal and re-seeding ability (Stem Cell Reports. 2015 Dec 8;5(6):979-987).

**Table 1**

| | All | -HGF | -NRG1 | -IGF1 |
|---|---|---|---|---|
| Total number of organoids (unit: 1×10⁵) | 22074.8 | 2495.7 | - | 20103.1 |
| PTK7 expression rate | 93.8% | 75.4% | - | 93.6% |
| Number of PTK7 expressing organoids (unit: 1×10⁵) | 20706.2 | 1881.7 | - | 18856.7 |

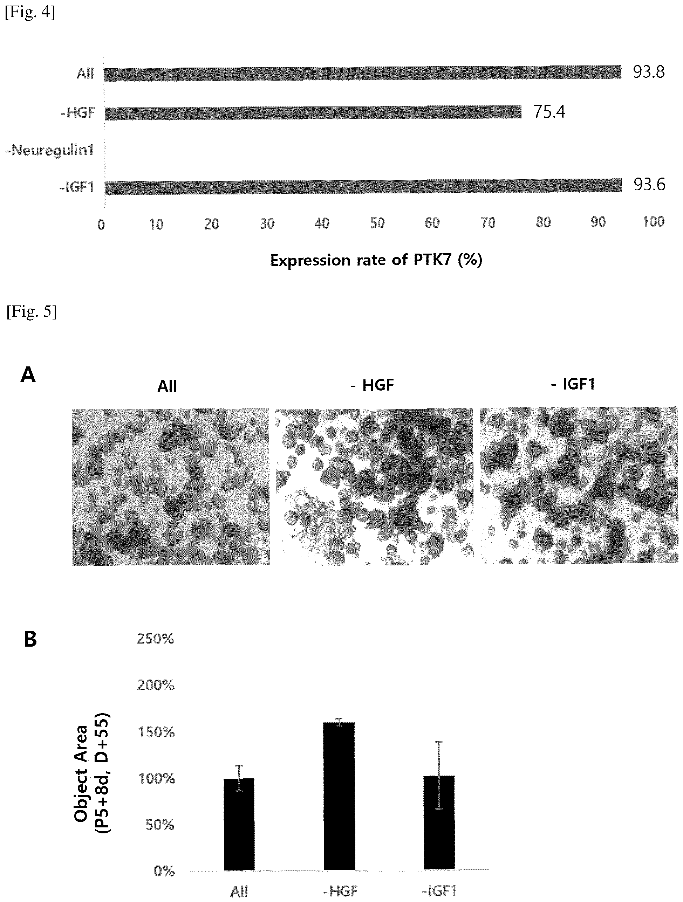

As a result, as shown in Table 1 and FIG. 4, it was confirmed that when using a medium not containing HGF ("-HGF"), the ratio of organoids expressing PTK7 among the total organoids prepared was about 75.4%. On the contrary, it was confirmed that when using a medium not containing IGF1 ("-IGF1"), the ratio of organoids expressing PTK7 among the total organoids prepared was about 93.6%, and the sternness was still maintained in most of the organoids prepared.

On the other hand, when using a medium not containing NRG1 ("-NRG1"), the expression level of stem cell markers was not analyzed because the organoids did not grow, and all died.

The above results suggest that HGF is an essential medium component in order to produce intestinal organoids that are not differentiated into normal cells even in long-term culture and still exhibit stemness.

### Example 2-3. Comparison of degree of differentiation

The degree of differentiation of intestinal organoids prepared with each medium composition according to Example 1 was compared.

In this Example, it was analyzed whether organoids lose sternness and were differentiated into normal cells during long-term culture (55 days in total, passage 5 and day 8). For this purpose, the Object Area, which refers to the size (perimeter) of the organoid, was measured. Basically, it can be analyzed that organoids grow when the object area increases, but it was determined that they were differentiated into normal cells under the following conditions. Organoids maintaining sternness proliferate in the form of a three-dimensional sphere, but as they differentiate, they form a three-dimensional budding shape. When fully differentiated, they attach to the culture container in a two-dimensional form and grow up. Since the size of the organoids attached to the culture container in the two-dimensional form is measured to be larger than that of the three-dimensional spherical organoids, it was determined that differentiation of organoids was induced when the object area increased above a certain standard. The object area was measured through the analysis program of Biotek equipment.

As a result, as shown in FIGs. 5A and 5B, when using a medium not containing HGF ("-HGF"), the object area of the organoid prepared was increased by about 1.5 times compared to that culturing in the medium containing all components ("All"). That is, it was confirmed that the medium not containing HGF had a higher degree of differentiation of intestinal organoids from stem cells into normal cells compared to other media. However, as the intestinal organoids growing attached to the culture container in a two-dimensional form are differentiated into aggregates that exhibit a radial shape., the object area does not increase infinitely (in FIG. 5A, the lower left side of the "-HGF" image showing the aggregates).

On the contrary, it was confirmed that when using a medium not containing IGF1 ("-IGF1"), the object area of the organoid prepared was similar to that of culturing in the medium containing all components ("All"). That is, it was confirmed that the medium not containing IGF1 had a lower degree of differentiation of intestinal organoids from stem cells into normal cells compared to other media.

On the other hand, when using a medium not containing NRG1 ("-NRG1"), the object area was not analyzed because the organoids did not grow and all died.

The above results suggest that HGF is an essential medium component in order to produce intestinal organoids that are not differentiated into normal cells even in long-term culture and still exhibit stemness.

Considering the results of Examples 2-1 to 2-3, it was found that a medium composition for preparing an intestinal organoid should comprise HGF and NRG1 as essential components in order to obtain a large number of intestinal organoids and maintain the sternness of the intestinal organoids even in long-term culture.

### Example 2-4. Karyotyping results

In order to confirm the genetic stability of the culture medium according to the present invention, karyotyping of the organoids was performed at certain passages during the culture period.

Specifically, the karyotype test was performed according to a method known in the art for an organoid cultured in passage 7, passage 9, passage 15 or passage 20.

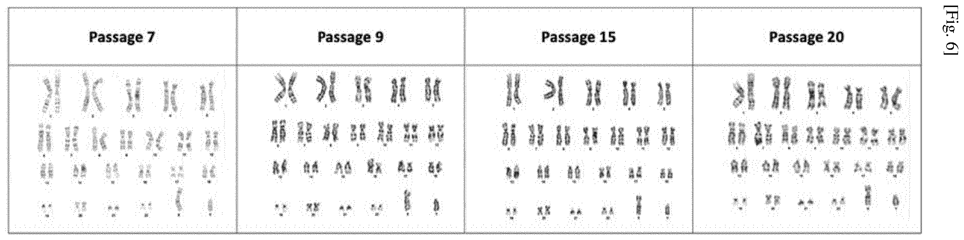

FIG. 6 shows the test results of organoids cultured in a medium containing all components ("All"). It was confirmed that there were no numerical or structural abnormalities or genetic alterations in chromosomes in organoids cultured at passage 7, 9, 15 or 20, and that they were not transformed into tumors.

The above results suggest that the medium comprising HGF, NRG1 and IGF1 does not have a negative genetic effect on the cultured organoids and allows long-term culture while maintaining genetic stability.

## Claims

1. A medium composition for use in preparing an intestinal organoid, comprising HGF and NRG1.

2. The composition according to claim 1, wherein the intestinal organoid expresses Tyrosine-protein kinase-like 7 (PTK7).

3. The composition according to claim 1, wherein the composition further comprises a basal medium.

4. The composition according to claim 3, wherein the basal medium is DMEM, MEM, BME, RPMI1640, F-10, F-12, α-MEM, GMEM, IMDM, DMEM/F12 or Advanced DMEM/F12.

5. The composition according to claim 1, wherein the composition further comprises one or more antibiotics selected from the group consisting of Gentamicin, Ampotericin and Streptomycin.

6. The composition according to claim 1, wherein the composition further comprises IGF1.

7. A method for preparing an intestinal organoid, comprising a step of culturing intestine-derived cells isolated from a subject using the medium composition according to claim 1.

8. An intestinal organoid prepared by the method according to claim 7.

9. A pharmaceutical composition for preventing or treating an intestinal disease, comprising the intestinal organoid according to claim 8.

10. The pharmaceutical composition according to claim 9, wherein the intestinal disease is one or more selected from the group consisting of inflammatory bowel disease, ulcer caused by inflammatory bowel disease and fistula caused by inflammatory bowel disease.

11. The pharmaceutical composition according to claim 10, wherein the inflammatory bowel disease is one or more selected from the group consisting of radiation colitis, radiation proctitis, ischemic colitis, intestinal Behcet's disease, Crohn's disease, ulcerative colitis and ulcerative proctitis.

12. The pharmaceutical composition according to claim 10, wherein the fistula is one or more selected from the group consisting of anal fistula and enterocutaneous fistula.

13. A method of evaluating the efficacy of a drug using the intestinal organoid according to claim 8.

14. The method according to claim 13, wherein the drug is a drug for preventing or treating an intestinal disease.

15. A drug efficacy evaluation system comprising the intestinal organoid according to claim 8.

16. A method of screening a drug using the intestinal organoid according to claim 8.

17. The method according to claim 16, wherein the drug is a drug for preventing or treating an intestinal disease.

18. A drug screening system comprising the intestinal organoid according to claim 8.
